# EUROPÄISCHE PATENTSCHRIFT

(11) **EP 0 909 942 B1**
(45) Veröffentlichungstag und Bekanntmachung des Hinweises auf die Patenterteilung: **25.09.2002**
(21) Anmeldenummer: 98203406.8
(22) Anmeldetag: 12.10.1998
(51) Int. Cl.: G01N 1/24, C21C 7/04, G01F 1/704

(54) **Vorrichtung zum Absaugen eines Messgases aus einem unter Vakuum stehenden Processgasraum**
Device for aspiration of a gas sample from a process-gas enclosure under vacuum
Appareil pour l'aspiration d'un échantillon gazeux d'une enceinte de gaz industriel sous vide

(30) Priorität: 16.10.1997 DE 19745808
(43) Veröffentlichungstag der Anmeldung: 21.04.1999
(73) Patentinhaber: Kuske GmbH, 47057 Duisburg (DE); Mannesmann AG, 40213 Düsseldorf (DE)
(72) Erfinder: Kuske, Günter, D-47057 Duisburg (DE); REIDICK Hermann Dipl.-Ing., D-46047 Oberhausen (DE); Clasen Klaus-D. Dipl.-Ing., D-47269 Duisburg (DE)
(74) Vertreter: Ackmann, Günther, Dr.-Ing.

(56) Entgegenhaltungen:
- EP-A- 0 511 583
- DE-A- 2 745 957
- DE-A- 3 604 206
- DE-A- 4 205 792
- US-A- 3 748 906
- US-A- 4 586 367
- US-A- 5 205 177

## Beschreibung

Die Erfindung betrifft ein Verfahren und eine Vorrichtung zur Probeentnahme aus einem Prozessgasraum zur Feststellung der Ausgasungen von flüssigem Stahl, wobei in einen Prozessgasraum eine Sonde mit einem Filter hineinragt und die Ausgasungen mittels einer trockenlaufenden, mehrstufigen Vakuumpumpe abgesaugt werden.

Bei der Herstellung von Stahl findet eine Vakuumentgasung des flüssigen Stahls Verwendung, um im Stahl enthaltene unerwünschte Einschlüsse zu entfernen oder zu verringern. Dabei wird beispielsweise eine mit flüssigem Stahl gefüllte Gießpfanne in eine Vakuumkammer gestellt und etwa 20 Minuten entgast. Für eine Überwachung des Entgasungsprozeßes wird eine Analyse des in der Vakuumkammer vorhandenen Gases angestrebt, die möglichst während des gesamten Entgasungsvorganges kontinuierlich stattfindet. Für Untersuchungszwecke ist es bekannt, das etwa 500°C heiße Meßgas aus dem Gasraum der Vakuumkammer mittels einer einem in der Regel transportablen Gasanalysegerät zugehörigen Vakuumpumpe abzusaugen. Diese Methode erlaubt jedoch nur eine diskontinuierliche Meßung und Gasanalyse. Aus der DE 28 39 315 A1 und der US 3 520 657 ist bekannt, aus dem Abgas der Vakuumanlage Meßgas abzusaugen, dieses zu analysieren und mit Hilfe der Analysedaten die Entgasung zu steuern. Hierfür ist ein in den Abgaskanal ragendes Saugrohr vorgesehen, das über einen außerhalb des Abgaskanals angeordneten Filter mit einer Vakuumpumpe verbunden ist, welche das abgesaugte Meßgas einem Analysegerät zuführt. Eine ähnliche Steuereinrichtung ist auch aus der DE 37 06 742 A1 bekannt, bei der ein von einer Vakuumpumpe aus der Abgasleitung abgesaugter Gasstrom zunächst durch eine Kühleinrichtung und eine Filtereinrichtung gesaugt und dann von dem von der Vakuumpumpe verdichteten Gasstrom ein Teilstrom mittels einer weiteren Förderpumpe über zusätzliche Filter unmittelbar dem Analysegerät zugeführt wird. Die aus diesen Druckschriften bekannten Absaugvorrichtungen haben sich jedoch in der Praxis nicht bewährt und erlauben keine ausreichend genaue Gasanalyse; insbesondere weil keine fortlaufend gleiche oder eine zu geringe Menge Gas aus dem Prozessgasraum abgeführt wird, wenn dieser von Atmosphärmdruck auf einen Unterdruck bis etwa 1 mbar gebracht wird. Die Vakuumpumpe muß aber nicht nur Meßgas aus einem derart hohen Unterdruckraum absaugen können, sondern auch geeignet sein, bei allen vorkommenden Druckverhältnissen eine gleiche Menge Gas während der gesamten Entgasungszeit abzusaugen und unmittelbar dem Analysegerät zuzuführen. Die in den Druckschriften hierfür vorgesehenen Vakuumpumpen sind hierfür nicht geeignet. Desweiteren werden die Filter leicht verstopft, wodurch die Gasströmung ebenfalls beeinträchtigt wird. Zu einer Verfälschung der Meßgaszusammensetzung kann es bei üblichen ölgeschmierten Vakuumpumpen durch in das Meßgas gelangende Öldämpfe u. dgl. kommen, aber auch bei einer eventuellen Kondensation von Gasbestandteilen, die bei diesen Unterdrücken in der vor dem Filter bereits abgekühlten Meßgasleitung erfolgen kann, wobei die Kondensationsprodukte an den Feststoffteilchen haften und im Filter zurückgehalten werden. Soweit bei den bekannten Absaugeinrichtungen Filter und Filterpatronen vorgesehen sind, ist zudem deren häufiger Austausch umständlich und aufwendig. Zur Gasentnahme aus einem Drehrohr-Zementofen ist in der DE 35 45 491 C2 eine Gasabsaugvorrichtung mit einer wassergekühlten Sonde und einem zugänglichen Filter beschrieben, der ebenfalls austauschbare Filterpatronen benötigt. Da aufgrund der Verbrennungs- und Umsetzungsprozesse im Zementdrehrohrofen an der Gasentnahmestelle Überdruck herrscht, ist eine solche Gasabsaugvorrichtung nicht zum Absaugen heißer Meßgase geeignet, die unter Vakuum stehen.

Die Erfindung geht weiterhin davon aus, dass in der DE 36 04 206 A1 eine mehrstufige, trocken laufende Drehschieber-Vakuumpumpe beschrieben ist, welche schmierölfrei betrieben wird und absolut ölfreie Gase und Produkte liefert. Die Verwendung dieser Vakuumpumpen zum Absaugen heißer Meßgase aus einem unter Vakuum stehenden Prozeßgasraum, insbesondere einer Vakuumkammer zur Entgasung von flüssigem Stahl ist bisher nicht bekannt und auch nicht ohne weiteres möglich.

Aus der deutschen Offenlegungsschrift 42 05 792 A1 ist eine Vorrichtung zur Probenentnahme aus den entstehenden Abgasen eines Verbrennungsprozesses im Hinblick auf die toxikologische und ökologische als besonders schädlich anzusehenden Substanzen PCDD und PCDF bekannt. Diese Probenentnahme findet unter zumindest Atmosphärendruck statt, wobei in dem offenbarten Verfahren ein abgesaugter Teilstrom des Abgases gasanalytisch über einen Filter und einen Absorber geleitet wird und anschließend quantitativ bestimmt werden kann. Hierzu ist es notwendig, dass das in der Katusche 10 eingeschlossene Filter- und Absorbermaterialien in aufwendiger Weise aufgeschlossen wird, wobei die Katusche aus dem Messsystem entfernt und durch eine neue ersetzt werden muss und zunächst nur eine Sammlung der Rückstände in der Katusche stattfindet, sodass keine kontinuierliche Probenentnahme mit einer sofortigen Gasanalyse möglich ist.

Der Erfindung liegt die Aufgabe zugrunde, ein Verfahren und eine Absaugvorrichtung gemäß dem des Anspruches 1 derart auszubilden, dass eine kontinuierliche Entnahme von Messgas während der gesamten Prozessdauer möglich ist. Außerdem soll die Absaugvorrichtung so beschaffen sein, dass während der Behandlungspausen eine Reinigung der Messungen durch Druckluftspülung möglich ist.

Diese Aufgabe wird durch das Verfahren mit den Merkmalen des Anspruchs 1 sowie durch die Vorrichtung mit den Merkmalen des Anspruchs 2 gelöst. Bei dieser sind mehrere Bauteile, nämlich Sonde, Filter, Vakuumpumpe und Rückspüleinrichtung in einer solchen Weise angeordnet und aufeinander abgestimmt, dass während der gesamten Prozeßdauer Meßgas aus dem Prozeßgasraum bei allen Druckverhältnissen im Prozeßgasraum, ohne Verfälschung der Zusammensetzung in einer konstanten Gasmenge abgesaugt und dem Gasanalysegerät zugeführt wird. Die Anordnung des Filters unmittelbar an der Sonde bewirkt zunächst, dass keine Feststoffteilchen größer als 4 µm mitgerissen werden und erst gar nicht in das Leitungssystem der Absaugvorrichtung gelangen. Kombiniert ist die Maßnahme mit einer Rückspüleinrichtung, durch welche der Filter in bestimmten Zeitabständen z. B. nach jedem Prozeß durch Rückspülung mit Druckluft gereinigt werden kann. Diese Maßnahme wiederum ist durch die Anordnung von Kugelhähnen in der Saugleitung und in der Anschlußleitung der Rückspülleitung ergänzt, die für den Rückspülvorgang einerseits die zur Vakuumpumpe führende Saugleitung absperrt und die Rückspülleitung anschließt und die in ihrem geöffneten Zustand einen praktisch ungeminderten Strömungsquerschnitt gewährleisten. Die Verwendung einer trocken laufenden mehrstufigen Vakuumpumpe hat zunächst den Vorteil, daß die Zusammensetzung des abgesaugten Meßgases durch keine Fremdstoffe, z. B. Dichtungsmittel, verfälscht wird. Erst eine solche mehrstufige Pumpe ist geeignet, um aus einem Prozessgasraum mit einem sehr niedrigen Gasdruck von etwa 1 mbar Meßgas abzusaugen. Da trocken laufende, mehrstufige Vakuumpumpen eine Wasserkühleinrichtung haben, kann die Kühlung so eingestellt werden, daß selbst bei den sehr niedrigen Drücken bis etwa 0,5 mbar keine Kondensation von Meßgasbestandteilen stattfinden kann.

Wegen der relativ hohen Temperatur des Prozessgases kommen für den Filter Textilgewebe u. dgl. nicht in Betracht. Als besonders geeignet hat sich ein Filter aus einem entsprechend feinen Metallgewebe oder einer metallischen Faserplatte erwiesen.

Durch eine Regelung der Drehzahl der Pumpe in Abhängigkeit von dem Druck im Prozessgasraum ist eine Entnahme von Meßgas während des gesamten Entgasungsvorganges erreichbar, das heißt auch während des Auf- und Abbaus des Vakuums. Somit ist eine fortlaufende Gasanalyse über den ganzen Prozesszeitraum möglich, die wiederum mittels entsprechender Einrichtungen bedarfsweise eine Steuerung oder Regelung des Prozesses zuläßt.

Um das Ansaugen von Leckgas zu verhindern, sind die in der Saugleitung bzw. in der Anschlußleitung der Rückspüleinrichtung angeordneten Kugelhähne in einem verschlossenen Gehäuse angeordnet, das über eine Rohrleitung an den Prozessgasraum angeschlossen ist.

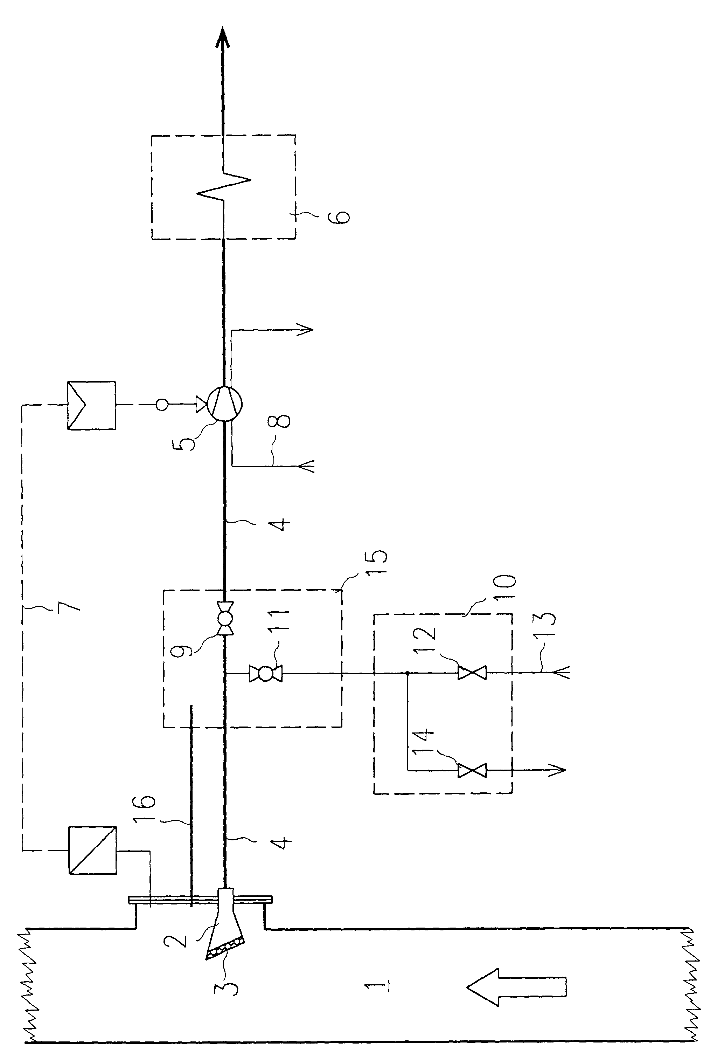

In der Zeichnung ist eine Vorrichtung zum Absaugen eines Meßgases aus einem unter Vakuum stehenden Prozessgasraum 1 schematisch dargestellt. Der Prozessgasraum kann beispielsweise die Vakuumkammer zur Entgasung von flüssigem Stahl sein, wobei eine mit flüssigem Stahl gefüllte Gießpfanne in die Vakuumkammer eingestellt wird. Nach dem Verschließen der Vakuumkammer wird diese mit einer Wasser- oder Dampfstrahlpumpe unter Vakuum gesetzt, wobei der Druck auf etwa 1 mbar erniedrigt wird. Der Entgasungsprozess dauert in der Regel etwa 20 Minuten. Das Prozessgas ändert während des Prozesses seine Zusammensetzung. Bei einer Stahlentgasung ist insbesondere der Anteil an O₂, CO, CO₂, N₂, H₂ und Ar von Interesse, wobei die Analyse z. B. mittels eines Massenspektrometers erfolgen kann.

Für eine kontinuierliche Gasanalyse wird dem Prozessgasraum fortlaufend Meßgas entnommen. Der Entnahme dient eine Sonde 2, die in den Prozessgasraum 1 hineinragt. Diese ist an ihrer Ansaugöffnung mit einem Filter 3 versehen, der temperaturbeständig und so ausgebildet ist, daß er Feststoffteilchen größer als 4 µm zurückhält. Geeignet ist beispielsweise ein etwa 1 bis 3 mm dickes Metallgewebe oder eine metallische Faserplatte. Das Filtermaterial muß so beschaffen sein, daß es einerseits die erwünschte Filterung herbeiführt, anderseits aber eine ausreichende Leitfähigkeit besitzt und sich durch Rückspülung leicht reinigen läßt, die in der Regel nach jedem Entgasungsvorgang stattfindet. Außerdem verläuft die Mündung der Sonde schräg, wobei der obere Teil weiter nach innen vorsteht und die Öffnung oben abdeckt. Hierdurch werden von dem Prozessgas gegebenenfalls nach oben mitgerissene und wieder nach unten fallende Feststoffteilchen gehindert, sich auf der Filterfläche abzulagern.

Wesentlicher Bestandteil der Absaugvorrichtung ist eine über eine Saugleitung 4 an die Sonde 2 angeschlossene Pumpe 5, die vom Typ einer trocken laufenden mehrstufigen Vakuumpumpe ist und einen Unterdruck bis etwa 0,5 mbar erzeugt. Die Pumpe 5 fördert das aus dem Prozessgasraum 1 abgesaugte Meßgas durch eine Meßgasaufbereitungseinrichtung, von der in der Zeichnung beispielsweise ein Meßgaskühler 6 dargestellt ist, zu einem Gasanalysegerät, z. B. einem Massenspektrometer. Die Drehzahl der Pumpe 5 wird durch eine Drehzahlregelvorrichtung 7 in Abhängigkeit von dem im Prozessgasraum 1 herrschenden Druck derart geregelt, daß fortlaufend eine gleich Menge Meßgas, z. B. zwei Liter pro Minute, abgesaugt wird. Die trocken laufende Vakuumpumpe ist als mehrstufige z. B. dreistufige Rotationskolbenmaschine ausgeführt, deren Drehkolben mit Dichtungsleisten versehen sind und daher trocken laufen, also keine Dichtungsflüssigkeit benötigen. Sie ist mit einer Wasserkühlung 8 versehen.

Um den Prozessgasraum 1 zwischen den Entgasungsprozessen gegenüber der Meßgasaufbereitungseinrichtung abzusperren und eine Reinigung der Sonde 2 vornehmen zu können, ist in der Saugleitung 4 zwischen der Sonde 2 und der Pumpe 5 ein Kugelhahn 9 angeordnet, der in der Offenstellung einen praktisch drosselfreien Strömungsquerschnitt aufweist. Außerdem ist an die Saugleitung 4 zwischen diesem Kugelhahn 9 und der Sonde 2 eine die Sonde 2 mit Druckluft beaufschlagende Rückspüleinrichtung 10 angeschlossen. Zwischen der Rückspüleinrichtung 10 und der Saugleitung 4 ist für Absperrzwecke ein weiterer Kugelhahn 11 vorhanden. Um das Ansaugen von Leckgas zu vermeiden, sind die Kugelhähne 9,11 zweckmäßig in einem verschlossenen Gehäuse 15 angeordnet, das über eine Rohrleitung 16 an den Prozessgasraum 1 angeschlossen ist, so daß diese Ventile von außen dem Prozessgasdruck unterliegen. Als Teile der Rückspüleinrichtung 10 sind ein dritter Kugelhahn 12, der an eine Druckluftleitung 13 anschließt, und ein der Entlüftung dienendes Absperrventil 14 dargestellt. Zur Reinigung der Sonde 2 wird der Kugelhahn 9 geschlossen, und nach Öffnen der Kugelhähne 11,12 wirkt die durch den Kugelhahn 12 erzeugte pulsierende Druckluft z. B. mit 10 bar auf den Filter 3 ein und spült diesen von festgesetzten Feststoffteilchen frei. Hierdurch bleibt der freie Strömungsquerschnitt des Filters 3 erhalten, der im Vakuumbereich besonders kritisch ist. Die Kugelhähne 9,11 und 12 werden durch eine elektronische Steuereinrichtung gestellt. Der Kugelhahn 12 wird so gesteuert, daß der Druck pulsiert, indem z. B. Druckluft- und Sperrphase mit je 10 Sekunden sechsmal aufeinander folgen, der Rückspülvorgang mithin etwa 120 Sekunden beträgt.

### Bezugszeichenliste

- 1: Prozessgasraum
- 2: Sonde
- 3: Filter
- 4: Saugleitung
- 5: Pumpe
- 6: Meßgaskühler
- 7: Drehzahlregelvorrichtung
- 8: Wasserkühlung
- 9: Kugelhahn
- 10: Rückspüleinrichtung
- 11: Kugelhahn
- 12: Kugelhahn
- 13: Druckluftleitung
- 14: Absperrventil
- 15: Gehäuse
- 16: Rohrleitung

## Patentansprüche

1. Verfahren zur Probeentnahme aus einem Prozessgasraum zur Feststellung der Ausgasungen von flüssigem Stahl, wobei in einem Prozessgasraum eine Sonde mit einem Filter hineinragt und die Ausgasungen mittels einer trockenlaufenden, mehrstufigen Vakuumpumpe abgesaugt werden,
**dadurch gekennzeichnet,**
**daß** die Meßgase aus einem unter Vakuum stehenden Prozessgasraum abgesaugt und über eine Meßgasaufbereitungseinrichtung unmittelbar einem Gasanalysegerät zugeführt werden, wobei die Sonde (2) mit einem temperaturbeständigen, Feststoffteilchen > 4 µm zurückhaltenden, Filter (3) versehen ist und wobei in der Saugleitung zwischen der Sonde (2) und der Vakuumpumpe (5) ein erster Kugelhahn (9) zur Trennung der Verbindung zwischen der Sonde (2) und der Vakuumpumpe (5) verwendet wird und über einen zweiten Kugelhahn (11) die Sonde mit Druckluft über eine Rückspüleinrichtung (10) beaufschlagt wird.

2. Vorrichtung zur Anwendung des Verfahrens nach Anspruch 1 zum Absaugen eines heißen Meßgases aus einem Prozessgasraum, insbesondere zur Entgasung von flüssigem Stahl, bestehend aus einer in den Prozessgasraum ragenden Sonde, einer das Meßgas durch eine Meßgasaufbereitungseinrichtung ansaugenden trockenlaufenden, mehrstufigen Vakuumpumpe und einem vom Meßgas durchströmten Filter,
**dadurch gekennzeichnet,**
**daß** der Prozessgasraum unter Vakuum steht und daß die Sonde (2) mit einem temperaturbeständigen, Feststoffteilchen größer als 4 µm zurückhaltenden Filter (3) versehen ist, daß in der Saugleitung (4) zwischen der Sonde (2) und der Vakuumpumpe (5) ein Kugelhahn (9) angeordnet ist, daß an die Saugleitung (4) zwischen der Sonde (2) und dem Kugelhahn (9) unter Zwischenschaltung eines weiteren Kugelhahns (11) eine die Sonde (2) mit Druckluft beaufschlagende Rückspüleinrichtung (10) angeschlossen ist und daß das Meßgas durch eine Meßgasaufbereitungseinrichtung einem Gasanalysegerät zugeführt wird.

3. Vorrichtung nach Anspruch 2,
**dadurch gekennzeichnet,**
**daß** der Filter (3) aus einem Metallgewebe oder einer metallischen Faserplatte besteht.

4. Vorrichtung nach Anspruch 2 oder 3,
**dadurch gekennzeichnet,**
**daß** die Drehzahl der Vakuumpumpe (5) in Abhängigkeit von dem Druck im Prozessgasraum (1) regelbar ist.

5. Vorrichtung nach einem der Ansprüche 2 bis 4,
**dadurch gekennzeichnet,**
**daß** die in der Saugleitung (4) und in der Zuleitung der Rückspüleinrichtung (10) angeordneten Kugelhähne (9,11) in einem geschlossenen Gehäuse (15) angeordnet sind, das über eine Rohrleitung (16) an den Prozessgasraum (1) angeschlossen ist.

## Claims

1. A method of taking, from a process gas chamber, a sample for determining the degassing of molten steel whereby a probe with a filter projects into a process gas chamber and the degassing products are extracted by a dry running, multi-stage vacuum pump, **characterised in that** the gas samples are extracted from a process gas chamber, which has a negative pressure, and are directed straight to a gas analyser via a gas sample processing unit whereby the probe (2) is equipped with a temperature resistant filter (3) retaining solid particles > 4 µm and whereby, in the suction line between probe (2) and vacuum pump (5), a first ball valve (9) is used for closing the connection between probe (2) and vacuum pump (5) and, via a second ball valve (11), compressed air is applied to the probe by a purging apparatus (10).

2. An apparatus for the application of the method according to claim 1 for extracting a hot gas sample from a process gas chamber, in particular for the degassing of molten steel, comprising a probe projecting into the process gas chamber, a dry running, multi-stage vacuum pump extracting the gas sample via a gas sample processing unit, and a filter through which the gas sample flows, **characterised in that** the process gas chamber is under vacuum and that probe (2) is equipped with a temperature resistant filter (3) retaining solid particles > 4 µm, that a ball valve (9) is located in the suction line (4) between probe (2) and vacuum pump (5), that a purging apparatus (10) is connected to suction line (4) between probe (2) and ball valve (9) via an additional ball valve (11), said purging apparatus applying compressed air to probe (2), and that the gas sample is directed to a gas analyser via a gas sample processing unit.

3. The apparatus according to claim 2, **characterised in that** filter (3) consists of a metallic fabric of a metallic fibre plate.

4. The apparatus according to claims 2 or 3, **characterised in that** the rotary speed of vacuum pump (5) can be controlled in relation to the pressure in process gas chamber (1).

5. The apparatus according to one of the claims 2 to 4, **characterised in that** the ball valves (9, 11), located in suction line (4) and the feed line of the purging apparatus (10), are mounted in an enclosed housing (15) which is connected to process gas chamber (1) via a pipe (16).

## Revendications

1. Procédé pour le prélèvement d'échantillons dans une chambre à gaz opératoire pour constater les émanations gazeuses d'acier liquide, une sonde avec un filtre étant insérée dans une chambre à gaz opératoire et les émanations gazeuses étant aspirées au moyen d'une pompe à vide à plusieurs échelons fonctionnant à sec,
**caractérisé en ce que**
les gaz à mesurer sont aspirés dans une chambre à gaz opératoire sous vide et acheminés directement par l'intermédiaire d'une installation de traitement des gaz à un appareil d'analyse gazeuse, la sonde (2) étant munie d'un filtre (3) résistant à la température, retenant les particules solides > 4 µm et un premier robinet hémisphérique (9) étant utilisé dans la conduite d'aspiration entre la sonde (2) et la pompe à vide (5) pour interrompre la liaison entre la sonde (2) et la pompe à vide (5) et la sonde étant sollicitée à l'air comprimé par un système de nettoyage (10) au moyen d'un deuxième robinet hémisphérique (11).

2. Procédé pour l'application du procédé de la revendication 1 pour l'aspiration d'un gaz brûlant à mesurer dans une chambre à gaz opératoire, en particulier pour le dégazage d'acier liquide, consistant en une sonde insérée dans la chambre à gaz opératoire, une pompe à vide à plusieurs échelons, fonctionnant à sec, aspirant le gaz à mesurer par l'intermédiaire d'une installation de traitement du gaz et en un filtre traversé par le gaz à mesurer,
**caractérisé en ce que**
la chambre à gaz opératoire est sous vide et que la sonde (2) est pourvue d'un filtre (3) résistant à la température, retenant les particules solides de taille supérieure à 4 µm, qu'un robinet hémisphérique (9) est installé dans la conduite d'aspiration (4) entre la sonde (2) et la pompe à vide (5), qu'à la conduite d'aspiration (4) entre la sonde (2) et le robinet hémisphérique (9), un système de nettoyage (10) sollicitant la sonde (2) à l'air comprimé est raccordé avec branchement intermédiaire d'un autre robinet hémisphérique (11) et que le gaz à mesurer est acheminé par l'intermédiaire d'une installation de traitement du gaz à un appareil d'analyse gazeuse.

3. Dispositif selon la revendication 2,
**caractérisé en ce que**
le filtre (3) est constitué d'une toile métallique ou d'une plaque métallique fibreuse.

4. Dispositif selon la revendication 2 ou 3,
**caractérisé en ce que**
le régime de la pompe à vide (5) est réglable en fonction de la pression dans la chambre à gaz opératoire (1).

5. Dispositif selon une des revendications 2 à 4,
**caractérisé en ce que**
les robinets hémisphériques (9, 11) installés dans la conduite d'aspiration et dans la conduite d'accès au système de nettoyage (10) sont disposés dans un boîtier fermé (15) qui est connecté par une canalisation (16) à la chambre à gaz opératoire (1).
